(19) Europäisches Patentamt
European Patent Office
Office européen des brevets

(11) EP 3 205 395 B1

(12) EUROPEAN PATENT SPECIFICATION

(45) Date of publication and mention of the grant of the patent:
**06.11.2019 Bulletin 2019/45**

(21) Application number: **15849085.4**

(22) Date of filing: **17.09.2015**

(51) Int Cl.:
***B01J 19/12*** *(2006.01)*
***C07C 249/06*** *(2006.01)*
***C07C 251/44*** *(2006.01)*
***C07D 225/02*** *(2006.01)*
***C07B 61/00*** *(2006.01)*
***F21V 3/00*** *(2015.01)*
***F21K 9/00*** *(2016.01)*
*F21Y 115/10* *(2016.01)*
*F21Y 107/30* *(2016.01)*

(86) International application number:
**PCT/JP2015/076415**

(87) International publication number:
**WO 2016/056370 (14.04.2016 Gazette 2016/15)**

# (54) PHOTOIRRADIATION DEVICE, PHOTOREACTION METHOD USING SAME, AND METHOD FOR PRODUCING LACTAM

LICHTBESTRAHLUNGSVORRICHTUNG, LICHTREAKTIVES VERFAHREN DAMIT UND VERFAHREN ZUR HERSTELLUNG VON LACTAM

DISPOSITIF DE PHOTO-IRRADIATION, PROCÉDÉ DE PHOTORÉACTION L'UTILISANT, ET PROCÉDÉ DE PRODUCTION DE LACTAME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2014 JP 2014208067**

(43) Date of publication of application:
**16.08.2017 Bulletin 2017/33**

(73) Proprietor: **Toray Industries, Inc.**
**Tokyo 103-8666 (JP)**

(72) Inventors:
- **UCHIUMI Ryota**
**Tokai-shi**
**Aichi 476-8567 (JP)**
- **TAKAHASHI Toru**
**Otsu-shi**
**Shiga 520-8558 (JP)**

(74) Representative: **Hoefer & Partner Patentanwälte mbB**
**Pilgersheimer Straße 20**
**81543 München (DE)**

(56) References cited:
**EP-A1- 2 695 879**
**WO-A1-2010/058607**
**WO-A1-2013/007815**
**WO-A1-2014/043964**
**CN-A- 103 994 342**
**GB-A- 1 010 151**
**GB-A- 1 017 242**
**JP-A- 2002 220 350**
**JP-A- 2005 288 288**
**JP-A- 2008 246 355**
**JP-A- 2010 006 775**
**JP-A- 2010 006 776**
**JP-A- 2012 089 755**
**JP-B1- S3 922 959**
**JP-B1- S4 413 498**
**US-A1- 2010 271 836**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 205 395 B1

## Description

### Technical Field of the Invention

The present invention relates to a photoirradiation device using light-emitting diodes as a light source, a photoreaction method using the photoirradiation device, and a method for producing a lactam by using the photoreaction method.

### Background Art of the Invention

A photoreaction is also called a photochemical reaction, and indicates the whole of chemical reactions each in which molecules are brought into a state having a high energy level, that is, a so-called excited state by photoirradiation, namely by making a radical reaction initiator absorb energy ascribed to the irradiated light, and the reaction is caused by the excited molecules. The photoreaction includes kinds of oxidation-reduction reaction by light, substitution-addition reaction by light, etc., and it is known that the applications include photo industry, copying technology, induction of photovoltaic power, and in addition, synthesis of organic compounds. Further, as unintentional photochemical reaction, photochemical smog and the like also belong to photochemical reaction.

For example, it is known that cyclohexanone oxime can be synthesized by photochemical reaction, and photonitrosation of cycloalkane is also a widely known technology at the present time. For instance, document JP 2010-6775 A discloses a method for producing a cycloalkanone oxime.

As light sources for the photoreaction which have been used so far, in most cases, a lamp, in which mercury, thallium, sodium or another metal is enclosed in an environment of vacuum or close to vacuum, voltage is applied, and the emitted electron beam is irradiated to the enclosed metal, and the light emitting ascribed to electric discharge in gas or vapor condition is utilized, for example, a discharge lamp or a fluorescent lamp, is used as the light source.

For example, in case where a high pressure mercury lamp is used as a light source, the effective wavelength is 365 nm to 600 nm. However, in this type of discharge lamp using mercury, specific light emission energy due to mercury exists also in the wavelength region including ultraviolet rays of less than 365 nm. Therefore, for example, in case of having light emission energy in a short wavelength region including ultraviolet rays of less than 350 nm, because it is comparable to the dissociation energy of many chemical bonds, a reaction other than the purpose proceeds and promotes a side reaction, and a brown tar-like deposit is formed on the photoirradiation surface of the discharge lamp, thereby reducing the yield. Therefore, in order to cut the ultraviolet rays, a water-soluble fluorescent agent or a UV-cut glass is used.

In order to reduce such problems in a mercury lamp and improve the luminous efficiency, it is known that a thallium lamp exhibiting light emission energy effective to a wavelength of 535 nm and a sodium lamp exhibiting light emission energy effective to a wavelength of 589 nm are effective. By using a sodium lamp as a light source, the yield is dramatically increased and a stable reaction becomes possible. Further, by using a high-pressure sodium discharge lamp, the industrially effective wavelength is set at 400 to 700 nm, and the efficiency can be increased in the wavelength region of 600 nm to 700 nm. The peak wavelength in this range can be estimated to be about 580 to 610 nm. However, in order to improve the electric properties and starting of the discharge lamp, coexistence of mercury is inevitable, and a filter for cutting ultraviolet rays due to mercury is necessary. In particular, short wavelengths less than 400 nm generated by mercury are unnecessary wavelengths because they have excessive energy and cause unnecessary side reactions.

Furthermore, the sodium lamp has a peculiar light emission energy peak in a wavelength region including infrared rays having a wavelength of 780 to 840 nm, and its energy intensity is frequently comparable to the maximum light emission energy of the sodium lamp. Since the dissociation energy of nitrosyl chloride is about 156 J/mol, which is comparable to the light emission energy at a wavelength near 760 nm according to Einstein's law, the light energy is small in the longer wavelength region and the nitrosyl chloride does not dissociate, and therefore, it does not contribute to a reaction and causes a great energy loss.

Light emitting diodes, also abbreviated as LEDs, have the advantage capable of converting electrical energy directly into light using semiconductors, and are attracting attention in terms of suppression of heat generation, energy saving, long life, and the like. Its history of development is still shallow, red LEDs were commercialized in 1962, LEDs such as blue, green and white were developed from around 2000, and they were commercialized for display and lighting uses. On the other hand, a discharge lamp used for a photoreaction has a very high output and a high luminous efficiency, but if it is attempted to obtain the light emission energy required for a photoreaction equivalent to that of a discharge lamp by LEDs, the required number of LEDs becomes enormous, and it has been considered that it is difficult to apply LEDs as a light source for a photoreaction, because problems in circuit design, LED heat countermeasure and cost remain. Furthermore, it is necessary to irradiate a reaction liquid with uniform light for the photoreaction, but the LED has a strong directivity and it is difficult to obtain the wavelength necessary for the reaction with a high efficiency, and also from this point of view, application of LEDs to the light source of the photoreaction has been considered to be

difficult. However, recently, as described in Patent document 1 and Patent document 2, there is an example in which photoreaction by LEDs is carried out using a small reaction apparatus, and moreover, as described in Patent document 2, solution of the problems for enlarging a light-emitting body is being in sight.

Prior art documents

Patent documents

Patent document 1: JP-A-2010-06776
Patent document 2: JP-A-2013-200944

Summary of the Invention

Problems to be solved by the Invention

However, in the inventions disclosed in these Patent documents 1 and 2, it cannot be said that a structure of a photoirradiation device capable of necessarily and effectively utilizing light linearly emitted from a light emitting diode is proposed. Further, if a photoreaction is aimed, in most case a target liquids is a flammable liquid, physical separation between the photoirradiation device as an ignition source and the target liquid is essential, however, in the inventions disclosed in Patent documents 1 and 2, there is a problem that light is obstructed by reflection by an obstacle for the separation provided in the photoirradiation route, that is, an optically transparent container, and the rate of the light transmission is reduced.

Accordingly, an object of the present invention is to provide a photoirradiation device capable of achieving a desired photoirradiation with a high optical transparency by suppressing reflection of light between a light-emitting diode light source and a target liquid isolated by an optically transparent container, a photoreaction method using the photoirradiation device, and a method for producing a lactam using the photoreaction method.

Means for solving the Problems

As a result of earnest investigation to solve the above-described problems, it has been found that by interposing a liquid phase having a specific refractive index in the photoirradiation route from the LED light source provided with an optically transparent container to a target liquid, desirably, by setting the irradiation angle of light from as LED narrow in radiation angle in a specific range with respect to the optically transparent container, the reflection of light at the surface of the optically transparent container, especially, the reflection of light generated when shifting from a high refractive index substance to a low refractive index substance, can be suppressed, and it is possible to efficiently perform the photoirradiation from the LED light source to the target liquid by suppressing energy loss ascribed to dissipation of light energy.

In order to solve the above-described problems, the present invention employs the following constitutions. Namely,

(1) A photoreaction method according to claim 1.
(2) The method according to (1), wherein the light emitting diodes are installed such that a center of optical axes of the light emitting diodes is set at 60° or less relatively to a normal line of an inner surface of the first optically transparent container.
(3) The method according to (1) or (2), wherein the liquid forming the liquid phase section is a nonflammable liquid.
(4) The method according to any one of (1) to (3), wherein the liquid forming the liquid phase section is water.
(5) The method according to any one of (1) to (4), wherein the second optically transparent container is disposed at least along an outer circumference of the first optically transparent container.
(6) The method according to any one of (1) to (5), wherein at least one of the first optically transparent container and the second optically transparent container is formed from a material having a refractive index of 1.4 or more.
(7) The method according to any one of (1) to (6), wherein at least one of the first optically transparent container and the second optically transparent container is formed from a glass.
(8) The method according to any one of (1) to (7), wherein the light-emitting body is covered with the gas phase section, and the gas phase section is formed from a gas having an oxygen concentration of 1% or less.
(9) The method according to (8), wherein the gas phase section is formed from an inert gas.
(10) The method according to (8) or (9), wherein the gas phase section is formed from nitrogen.

(11) The photoreaction method according to (10), wherein the destination of photoirradiation is a liquid, and the composition of the liquid contains at least a carbon atom.

(12) The photoreaction method according to (11), wherein the liquid as the destination of photoirradiation is a cycloalkane.

(13) The photoreaction method according to (12), wherein a cycloalkanone oxime is produced by performing photoirradiation to the cycloalkane and a photo nitrosating agent.

(14) The photoreaction method according to (13), wherein the cycloalkanone oxime is cyclohexanone oxime or cyclododecanone oxime.

(15) The photoreaction method according to (13) or (14), wherein the photo nitrosating agent is nitrosyl chloride or trichloronitrosomethane.

(16) A method for producing a lactam characterized by using a cycloalkanone oxime produced by the photoreaction method according to any one of (13), to (15).

## Effect according to the Invention

In the present invention, by interposing the liquid phase section, formed from the liquid having a refractive index closer to that of the first optically transparent container than that of the gas forming the gas phase section inside the first optically transparent container, between the first and second optically transparent containers provided for separating the light emitting body using the LEDs as a light source and the reaction liquid as the target liquid, the reflection of the light irradiated from the LED light source to the target liquid can be efficiently suppressed. Further, if the incident angle of the light to the inner surface of the first optically transparent container is set small, the reflection of the light can be suppressed more efficiently. Thus, the optical transparency can be increased by suppressing the reflection of the light, the optical irradiation to the target liquid can be carried out without accompanying with a great energy loss, and it becomes possible to obtain high reaction yield and reaction amount in the photochemical reaction. In particular, the present invention is extremely useful for the photoreaction for producing cycloalkanone oxime and the production of lactam using the cycloalkanone oxime.

## Brief explanation of the drawings

Fig. 1 is a schematic vertical sectional view of a photoirradiation device according to an embodiment of the present invention.

Fig. 2 is an enlarged schematic cross-sectional view of the photoirradiation device shown in Fig. 1.

Fig. 3 is a schematic partial perspective view of a light-emitting body shown in Fig. 2.

Fig. 4 is a schematic elevational view of a substrate shown in Fig. 3.

Fig. 5 is an enlarged cross-sectional view showing an example of the photoirradiation in the photoirradiation device shown in Fig. 1.

Fig. 6 is a schematic diagram showing an example of refraction of light at an interface between media.

Fig. 7 is a schematic diagram showing another example of refraction of light at an interface between media.

## Embodiments for carrying out the Invention

Hereinafter, embodiments of the present invention will be explained referring to figures.

In the present invention, the photoirradiation device is a device which has a light-emitting body emitting light and can irradiate light to an object. The object is, for example, a reaction liquid becoming a raw material as a target liquid of photoreaction. Figs. 1 to 4 show a photoirradiation device according to an embodiment of the present invention, and in particular, show an example in case where light from the photoirradiation device is irradiated to a reaction liquid as a target liquid. In the example shown in Fig. 1 and Fig. 2, the photoirradiation device 1 is inserted in a reaction vessel 2 so as to be used for the photoreaction of the reaction liquid 3 in the reaction vessel 2. The photoirradiation device 1 is provided with a power supply unit 4 at its upper portion and a light-emitting body 6 equipped with a large number of light emitting diodes (hereinafter, also abbreviated as LEDs) 5 (shown in Fig. 2) at its lower portion in the mounting posture shown in Fig. 1, and the upper side of the power supply unit 4 is sealed with a lid 7. The light-emitting body 6 is covered with a first optically transparent container 8, and in this embodiment, the inside of the first optically transparent container 8, that is, the portion between the light-emitting body 6 and the first optically transparent container 8 is formed as a gas phase section 9. A second optically transparent container 10 is provided outside the first optically transparent container 8, and the layer between the first optically transparent container 8 and the second optically transparent container 10 is formed as a liquid phase section 11. This liquid phase section 11 is formed from a liquid closer to the refractive

index of the first optically transparent container 8 than gas, and for example, it is formed by filling nonflammable liquid between the first and second optically transparent containers 8 and 10.

The light emitting diode is a light emitting diode which emits ultraviolet rays, visible light and infrared rays, and as the light emitting diode 5 to be used, a type selected for the wavelength required for the application of the photoirradiation device 1 can be appropriately selected. The shape and size of the light emitting diode 5 is not particularly limited, and it is possible to use a shape and size depending upon the purpose. Although any of bullet type, mounting type, chip type and the like may be used, one capable of radiating heat from the back side of the light emitting diode 5 is preferable because heat removal is easy. Further, one provided with a heat radiation substrate on the back surface of the light emitting diode 5 is desired because a large heat transfer area can be obtained and the cooling performance is improved.

In the present invention, the light-emitting body means a portion having a surface emitting light in the photoirradiation device. Concretely, for example, a plurality of light emitting diodes 5 are mounted on a planar substrate 12 as shown in Fig. 4, and the planar substrate 12 is attached, for example, to each exterior surface of a structural body 13 whose cross-sectional shape is a star-shaped hexadecagon as shown in Figs. 2 and 3, whereby it is possible to irradiate light toward the outside.

In this case, the shape of the light-emitting body 6 is decided by the shape pf the structural body 13 arranged with the above-described planar substrates 12. However, the shape of the light-emitting body 6 is not particularly restricted, and, in embodiments not according to the invention, as the cross-sectional shape, except the above-described star shape, a circular shape, a polygonal shape and the like can be employed. Further, by changing the cross-sectional shape of the light-emitting body 6, the area of the light emitting diode placement surface changes, the possible number of mounted light emitting diodes can be increased and decreased, and the shape can be decided depending upon the purpose.

The first optically transparent container 8 is a container covering the light-emitting body 6 and is provided to protect the light-emitting body 6 from the outside. This first optically transparent container 8 may be formed by using a material transmitting light. If a raw material having transmission wavelength selectivity is used for the optically transparent container, it is possible to suppress transmission of unnecessary wavelength light. As the overall shape of the first optically transparent container 8, for example, a test tube type can be exemplified as shown in Fig. 1, but the shape is not particularly restricted, and cylinder type, box type, spherical type and the like can be selected appropriately depending upon the purpose.

The second optically transparent container 10 is a container disposed outside the first optically transparent container 8, and may be formed by using a material transmitting light similarly to the first optically transparent container 8. This material of the second optically transparent container 10 may be the same as or different from that of the first optically transparent container 8. As the shape thereof, for example, a test tube type can be exemplified as shown in Fig. 1, but the shape is not particularly restricted, and cylinder type, box type, spherical type and the like can be selected appropriately depending upon the purpose, and further, it may have a similar shape or a different shape to the first optically transparent container 8.

In the present invention, the liquid phase section formed from a liquid closer to the refractive index of the first optically transparent container than that of the gas forming the gas phase section inside the first optically transparent container means a portion formed from a liquid whose difference in refractive index from the first optically transparent container is smaller than that of the above-described gas, and is, for example, a portion formed by being filled with such a liquid. In this liquid phase section, the liquid may be flowed therethrough at all times or it may be enclosed. As shown in Figs. 1 and 2, the liquid phase section 11 is interposed between the first optically transparent container 8 and the second optically transparent container 10.

In case where a liquid, for example, water is flowed through the liquid phase section 11, by lowering the temperature of the flowing water, the refractive index of water can be enhanced and the difference in refractive index from the first optically transparent container can be further reduced. Further, except water, a nonflammable liquid and a liquid having a small difference in refractive index from the first optically transparent container can be used depending upon the purpose. Furthermore, depending upon the purpose, a liquid inert to water may be added and used. It is possible to change the refractive index by the addition.

In the present invention, by interposing the above-described liquid phase section 11 between the first optically transparent container 8 and the second optically transparent container 10, the energy loss ascribed to reflection in the irradiation route of the light from the light-emitting body 6 is suppressed.

Using Fig. 6 and Fig. 7, refraction and reflection of light at an interface between media 1 and 2 with refractive indexes n1 and n2 different from each other will be explained. Light produces reflection loss at the interface between the media with different refractive indexes. The surface reflectance of light is represented by the following equation (1), and it is known that the larger the difference in refractive index between the media forming the interface is, the greater the reflection loss is. Where, $\theta 1$ indicates an incident angle and $\theta 2$ indicates a refractive angle.

$$\text{Surface reflectance } R=0.5 \times \{\tan(\theta1-\theta2)/\tan(\theta1+\theta2)\}^2 + \{\sin(\theta1-\theta2)/\sin(\theta1+\theta2)\}^2 \quad (1)$$

Namely, in case where there is a media interface having a large difference in refractive index on the optical path, loss ascribed to light reflection increases. Accordingly, in the present invention, by interposing the liquid phase section 11 formed from a liquid having a difference in refractive index as small as possible, in the space between the first optically transparent container 8 and the second optically transparent container 10, the light energy loss ascribed to reflection is reduced.

For example, an example of an optical path accompanied by refraction and reflection when the photoirradiation is performed by the photoirradiation device 1 of Fig. 1 is shown in Fig. 5. As shown in Fig. 5, the incident light, which is irradiated along the optical axis 21 of the light emitting diodes 5 and is entered at a predetermined incident angle 23 relatively to a normal line 22 of the inner surface of the first optically transparent container 8, passes through the interior of the material of the first optically transparent container 8 after being refracted at the inner surface of the first optically transparent container 8, is entered at an incident angle 25 relatively to a normal line 24 of the interface between the outer surface of the first optically transparent container 8 and the liquid phase section 11, passes through the interior of the liquid phase section 11 after being refracted at the interface, passes through the interior of the material of the second optically transparent container 10 after being refracted at the interface between the liquid phase section 11 and the second optically transparent container 10, and after being refracted at the outer surface of the second optically transparent container 10, it is irradiated to the reaction liquid 3 as a transmitted light 26. In the illustrated example, at the interface between the gas phase section 9 and the first optically transparent container 8, at the interface between the first optically transparent container 8 and the liquid phase section 11, at the interface between the liquid phase section 11 and the second optically transparent container 10 and at the interface between the second optically transparent container 10 and the reaction liquid 3, respectively, the reflected lights 27 are generated, and by forming the liquid phase section 11 with a liquid having a difference in refractive index from the first optically transparent container 8 smaller than that of gas, light energy loss ascribed to reflection can be reduced. In particular, as compared with a case where the liquid phase section 11 is supposed to be formed from a gas, the incident angle of the light passing through the interior of the liquid phase section 11 and entering into the second optically transparent container 10 relative to the normal line of the container surface can be suppressed small, and therefore, at the interface between the first optically transparent container 8 and the liquid phase section 11 and at the interface between the liquid phase section 11 and the second optically transparent container 10, light energy loss ascribed to reflection can be reduced.

Here, the optical axis 21 of the light emitting diodes 5 means an imaginary center line of a light flux emitted from the light emitting diodes 5 mounted on the aforementioned planar substrate. For example, in case where a plurality of light emitting diodes are equidistantly arranged in a planar substrate and light is irradiated, the optical axis becomes a line extending in the direction perpendicular to the planar substrate from the center of gravity of the planar substrate.

The incident angle of light into the container surface is determined by the positional relationship between the light emitting surface on which the light emitting diodes 5 are disposed and the container surface, concretely, the angle formed between the light emitting surface and the container surface. Since the angle formed between the light emitting surface and the container surface is determined by the shape of the structural body 13 in which the light emitting diodes 5 are disposed and the shape of the container, the incident angle can be controlled by adjusting the shapes of the structural body 13 and the container.

According to the aforementioned equation (1), the reflectance of light varies depending on the incident angle, the smaller the incident angle is, the smaller the reflectance is, and the loss of light can be reduced.

The incident angle into the first optically transparent container 8 is preferably 0° or more and 60° or less, and more preferably 0° or more and 45° or less.

Namely, it is preferred that the optical axis of the light-emitting diodes 5 is set at 45° or less relatively to the normal line of the inner surface of the first optically transparent container 8. This is to arrange the light emitting diodes 5 so that the angle formed by the center of the optical axis and the normal line of the surface of the optically transparent container 8, that is, the incident angle of light into the surface of the optically transparent container 8, becomes 45° or less.

Furthermore, it is known that, when light is entered from the side of a high refractive index medium to the side of a low refractive index medium, total reflection, in which all of the incident lights are not transmitted, is caused at a certain incident angle or more. The minimum incident angle causing total reflection is called as a critical angle, and the critical angle is represented by the following equation (2) using the refractive index n1 of the incident side medium and the refractive index n2 of the transmission side medium. Light entered at an incident angle of the critical angle or more is not transmitted but is all reflected at the interface. Therefore, it is important for suppressing the reflection loss to set so that the incident angle does not exceed the critical angle. Here:

$$\text{Critical angle } \theta c = \arcsin(n2 / n1) \qquad (2)$$

Thus, effectively transmitting the light irradiated from the light-emitting body 6 to the reaction liquid 3 present outside the two optically transparent containers 8 and 10 can be achieved by filling the space between the first optically transparent container 8 and the second optically transparent container 10 with the medium having a refractive index close to that of the optically transparent container and making the incident angle to the container surface small.

In the present invention, it is preferred to use a liquid which forms the liquid phase section 11 as the above-described medium having a refractive index close to that of the optically transparent container. Where, in the present invention, a nonflammable liquid means a liquid that does not correspond to a dangerous substance specified by the Fire Services Act and is a liquid that transmits light. Concretely, exemplified are ethylene glycol 50% aqueous solution, silicone oil, water and the like.

The difference in refractive index between the liquid forming the liquid phase section 11 and the first optically transparent container 8 is preferably smaller than the difference in refractive index between the optically transparent container and air, and the liquid forming the liquid phase section 11 may be one in which the refractive index is adjusted by dissolving a soluble substance in water such as one in which sugar or the like is dissolved in water or one an aqueous glycerin solution. Further, since the refractive index varies depending upon temperature, the refractive index may be adjusted by adjusting the temperature.

Further, by adding a substance which absorbs light having a specific wavelength to the above-described liquid, light of a specific wavelength unnecessary for the reaction may be removed.

In the present invention, it is preferred that at least one of the first and second optically transparent containers is formed from a material having a refractive index of 1.4 or more. As such a material, any of an organic material typically represented by a resin or an inorganic material typically represented by a glass may be employed. More concretely, acrylic resin, methacrylic resin, polycarbonate, polystyrene, polyvinyl chloride, polyester, borosilicate glass, soda-lime-silica glass, lead glass and the like can be exemplified.

The refractive index of a nonflammable liquid such as water filled in the liquid phase section is generally smaller than the refractive index of the optically transparent solid. In particular, since a material that reduces the difference in refractive index from the liquid phase section is preferred, for forming the optically transparent container, it is more preferred to use borosilicate glass having a low refractive index among optically transparent solids and having high pressure resistance and chemical stability.

A material which absorbs light having a specific wavelength may be used for the first and second optically transparent containers. By making the containers absorb the wavelength unnecessary for photoreaction, only the wavelength necessary for photoreaction can be transmitted to the reaction liquid.

In the present invention, the phrase "the light-emitting body 6 is covered with the gas phase section 9" means a state where the space between the light-emitting body 6 and the first optically transparent container 8 is filled with gas.

In case where the above-described gas phase section 9 contains oxygen, it causes oxidation deterioration of electronic components such as the light emitting diodes 5. Therefore, in order to lengthen the lifetime of the light-emitting body 6, it is desired that the gas has an oxygen concentration of 2% or less, more preferably 1.5% or less.

As the gas forming the gas phase section 9, an inert gas can be used, and rare gases such as helium, neon, argon, krypton and xenon can be exemplified, but it is preferred to use nitrogen as an inert gas which can be easily and inexpensively obtained. With respect to this gas phase section 9, the gas may be constantly flown or may be in a enclosed condition.

In the present invention, the destination of the photoirradiation may be a liquid which contains carbon atoms. Namely, the photoirradiation device according to the present invention can be used as a light source for photoreaction, and at least one destination of the photoirradiation may be a raw material system composed of a liquid. The liquid served as a raw material is not particularly restricted as long as it is a liquid containing carbon atoms, and for example, hydrocarbons such as alkane and cycloalkane can be exemplified.

In the present invention, although the cycloalkane is not particularly limited in the number of carbon atoms, for example, preferred are cyclopropane, cyclobutane, cyclopentane, cyclohexane, cycloheptane, cyclooctane, cyclononane, cyclodecane, cycloundecane, and cyclododecane. In particular, cyclohexane as a raw material of ε-caprolactam and cyclododecane as a raw material of ω-lauryllactam are preferred.

Using the above-described cycloalkane and photo nitrosating agent, cycloalkanone oxime is obtained by photochemical reaction due to the photo irradiation of light emitting diodes. As the photo nitrosating agent, for example, nitrosyl chloride or a mixed gas of nitrosyl chloride and hydrogen chloride is preferable. Besides, since any of the mixed gas of nitric monoxide and chlorine, the mixed gas of nitric monoxide, chlorine and hydrogen chloride, the mixed gas of nitrose gas and chlorine, etc. acts as nitrosyl chloride in the photoreaction system, it is not limited to these supply forms of the nitrosating agent. Further, trichloronitrosomethane obtained by photochemical reaction of nitrosyl chloride and chloroform may be used as a nitrosating agent. When the photochemical reaction is carried out in the presence of

hydrogen chloride, the cycloalkanone oxime becomes its hydrochloride, but it may be in the form of hydrochloride as it is.

By the above-described photoreaction, it is possible to obtain cycloalkanone oxime which depends upon the carbon number of the cycloalkane. For example, cyclohexanone oxime is obtained by phot nitrosating reaction with nitrosyl chloride using cyclohexane. Further, cyclododecanone oxime is obtained by photo nitrosating reaction with nitrosyl chloride using cyclododecane.

<Method for producing lactam>

A photochemical reaction is carried out using the photoirradiation device according to the present invention, and a lactam is obtained by Beckmann rearrangement of the obtained cycloalkanone oxime. For example, in the reaction of Beckmann rearrangement of cyclohexanone oxime, ε-caprolactam is obtained as shown by the following reaction formula [Chemical formula 1]. Further, ω-laurolactam is obtained in the reaction of Beckmann rearrangement of cyclododecanone oxime.

[Chemical formula 1]

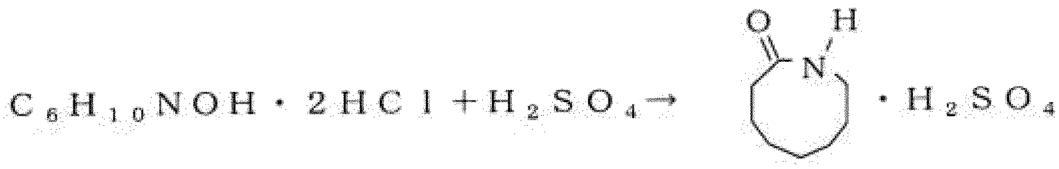

Examples

Hereinafter, the present invention will be explained in more detail with reference to Examples.

Example 1, Reference Example 1:

For the light emitting body using light emitting diodes as a light source, borosilicate glass with a refractive index of 1.49 was used for the first optically transparent container, the gas phase section was formed from $N_2$, and water was placed in the liquid phase section covering the first optically transparent container, and the transmittance of light (optical transparency) from the first optically transparent container to the liquid phase section, in case where the incident angle from the first optically transparent container to the interface between the container outer surface and the liquid phase section was changed, was calculated. As the results are shown in Table 1, high transmittance was obtained. In case where water was placed in the liquid phase section, total reflection occurred at an incident angle of 70 degrees or more (Reference Example 1).

Comparative Example 1, Reference Comparative Example 1:

Transmittance was calculated in the same manner as in Example 1 other than a condition where $N_2$ was filled in the liquid phase section. The results are shown in Table 1. The transmittance became lower as compared with the case where water was placed in the liquid phase section. In case where $N_2$ was filled in the liquid phase section, total reflection occurred at an incident angle of 50 degrees or more (Reference Comparative Example 1).

[Table 1]

| | Incident angle from inside of container to outer surface of container (liquid phase section) (°) | Incident side medium | | Refractive side medium | | Reflectance (-) | Optical transparency from first optically transparent container to liquid phase section (-) | Remark |
|---|---|---|---|---|---|---|---|---|
| | | Optically transparent container | | Liquid phase section | | | | |
| | | Material | Refractive index | Material | Refractive index | | | |
| Example 1 | 10 | borosilicate glass | 1.49 | water | 1.33 | 0.00322 | 0.9968 | |
| | 20 | | | | | 0.00329 | 0.9967 | |
| | 30 | | | | | 0.00371 | 0.9963 | |
| | 40 | | | | | 0.00563 | 0.9944 | |
| | 50 | | | | | 0.01560 | 0.9844 | |
| | 60 | | | | | 0.12237 | 0.8776 | |
| Reference Example 1 | 70 | | | | | 1.00000 | 0.0000 | total reflection |
| | 80 | | | | | 1.00000 | 0.0000 | total reflection |
| Comparative Example 1 | 10 | | | $N_2$ | 1.00 | 0.03880 | 0.9612 | |
| | 20 | | | | | 0.04037 | 0.9596 | |
| | 30 | | | | | 0.05306 | 0.9469 | |
| | 40 | | | | | 0.21842 | 0.7816 | |
| Reference Comparative Example 1 | 50 | | | | | 1.00000 | 0.0000 | total reflection |
| | 60 | | | | | 1.00000 | 0.0000 | total reflection |
| | 70 | | | | | 1.00000 | 0.0000 | total reflection |
| | 80 | | | | | 1.00000 | 0.0000 | total reflection |

Further, in case where water is placed in the liquid phase section, the critical angle at which total reflection occurs becomes larger than the case where $N_2$ is filled, and therefore, it is possible to use light more effectively.

Example 2:

With respect to the light emitting body using light emitting diodes as a light source, $N_2$ was used for the gas phase section covering the light emitting diodes, and borosilicate glass with a refractive index of 1.49 was used for the first optically transparent container. The incident angle of the light on the optical axis of the light emitting diodes from the gas phase section to the inner surface of the first optically transparent container was set at 10°, and the transmittance of light from the gas phase section to the first optically transparent container was calculated. The results are shown in Table 2.

Examples 3 to 9:

The optical transparency (transmittance of light) was calculated in the same manner as in Example 2 other than a condition where the incident angle was changed from 20° to 80°. The results are shown in Table 2.

[Table 2]

| | Incident angle from gas phase section to inner surface of container (°) | Incident side medium | | Refractive side medium | | Reflectance (-) | Optical transparency from gas phase section to first optically transparent container (-) |
|---|---|---|---|---|---|---|---|
| | | Gas phase section | | Optically transparent container | | | |
| | | Material | Refractive index | Material | Refractive index | | |
| Example 2 | 10 | $N_2$ | 1.00 | borosilicate glass | 1.49 | 0.039 | 0.9613 |
| Example 3 | 20 | | | | | 0.039 | 0.9610 |
| Example 4 | 30 | | | | | 0.040 | 0.9598 |
| Example 5 | 40 | | | | | 0.044 | 0.9556 |
| Example 6 | 50 | | | | | 0.056 | 0.9438 |
| Example 7 | 60 | | | | | 0.088 | 0.9125 |
| Example 8 | 70 | | | | | 0.169 | 0.8308 |
| Example 9 | 80 | | | | | 0.386 | 0.6140 |

Industrial Applicability

The photoirradiation device according to the present invention is applicable to any field in which efficient photoirradiation is desired, and in particular, it is suitable to a photoreaction method using the photoirradiation device and a method for producing a lactam using the photoreaction method .

Explanation of symbols

1: photoirradiation device

2: reaction vessel
3: reaction liquid
4: power supply unit
5: light emitting diode
6: light-emitting body
7: lid
8: first optically transparent container
9: gas phase section
10: second optically transparent container
11: liquid phase section
12: substrate
13: structural body
21: optical axis
22: normal line of inner surface of first optically transparent container
23: incident angle to container inner surface
24: normal line of container outer surface
25: incident angle to container outer surface
26: transmitted light
27: reflected light

## Claims

1. A photoreaction method using a photoirradiation device (1), wherein a first optically transparent container (8) for covering a light-emitting body (6) is provided, and on the outside thereof, a liquid phase section (11) formed from a liquid having a refractive index closer to that of said first optically transparent container (8) than that of a gas forming a gas phase section (9) inside said first optically transparent container (8), and a second optically transparent container (10) for covering said liquid phase section (11) are provided, wherein the photoirradiation device (1) is inserted in a reaction vessel (2), the reaction vessel (2) further comprising a reaction liquid (3), wherein said liquid comprises a cycloalkane, **characterized in that** the photoirradiation device (1) uses light emitting diodes (5) as a light source, and **in that** the light-emitting body (6) is provided with said light emitting diodes (5), wherein the light-emitting body (6) comprises a plurality of light emitting diodes (5) mounted on a planar substrate (12), and wherein the planar substrate (12) is attached to each exterior surface of a structural body (13) whose cross-sectional shape is a star-shaped hexadecagon, whereby it is possible to irradiate light toward the outside.

2. The photoreaction method according to claim 1, wherein said liquid forming said liquid phase section (11) is a nonflammable liquid.

3. The photoreaction method according to claim 1 or 2, wherein said liquid forming said liquid phase section (11) is water.

4. The photoreaction method according to any one of claims 1 to 3, wherein said second optically transparent container (10) is disposed at least along an outer circumference of said first optically transparent container (8).

5. The photoreaction method according to any one of claims 1 to 4, wherein at least one of said first optically transparent container (8) and said second optically transparent container (10) is formed from a material having a refractive index of 1.4 or more.

6. The photoreaction method according to any one of claims 1 to 5, wherein at least one of said first optically transparent container (8) and said second optically transparent container (10) is formed from a glass.

7. The photoreaction method according to any one of claims 1 to 6, wherein said light-emitting body (11) is covered with said gas phase section (9), and said gas phase section (9) is formed from a gas having an oxygen concentration of 1% or less.

8. The photoreaction method according to claim 7, wherein said gas phase section (9) is formed from an inert gas.

9. The photoreaction method according to claim 7 or 8, wherein said gas phase section (9) is formed from nitrogen.

**10.** The photoreaction method according to claim 1, wherein a cycloalkanone oxime is produced by performing photoirradiation to said cycloalkane and a photo nitrosating agent.

**11.** The photoreaction method according to claim 10, wherein said cycloalkanone oxime is cyclohexanone oxime or cyclododecanone oxime.

**12.** The photoreaction method according to claim 10 or 11, wherein said photo nitrosating agent is nitrosyl chloride or trichloronitrosomethane.

**13.** A method for producing a lactam **characterized by** using a cycloalkanone oxime produced by the photoreaction method according to any one of claims 10 to 12.

**Patentansprüche**

**1.** Lichtreaktives Verfahren unter Verwendung einer Lichtbestrahlungsvorrichtung (1), wobei ein erster optisch transparenter Behälter (8) zum Abdecken eines Licht emittierenden Körpers (6) vorgesehen ist, und an der Außenseite davon ein Flüssigphasenabschnitt (11), der aus einer Flüssigkeit gebildet ist, deren Brechungsindex näher an dem des ersten optisch transparenten Behälters (8) liegt als an dem eines Gases, das einen Gasphasenabschnitt (9) innerhalb des ersten optisch transparenten Behälters (8) bildet, und ein zweiter optisch transparenter Behälter (10) zum Abdecken des Flüssigphasenabschnitts (11) vorgesehen sind, wobei
die Lichtbestrahlungsvorrichtung (1) in ein Reaktionsgefäß (2) eingesetzt ist, wobei das Reaktionsgefäß (2) ferner eine Reaktionsflüssigkeit (3) aufweist, wobei die Flüssigkeit ein Cycloalkan aufweist,
**dadurch gekennzeichnet, dass** die Lichtbestrahlungsvorrichtung (1) Leuchtdioden (5) als Lichtquelle verwendet, und dass der Licht emittierende Körper (6) mit den Leuchtdioden (5) versehen ist, wobei der Licht emittierende Körper (6) eine Vielzahl von Leuchtdioden (5) aufweist, die auf einem ebenen Substrat (12) montiert sind, und wobei das ebene Substrat (12) an jeder Außenfläche eines Strukturkörpers (13) angebracht ist, dessen Querschnittsform ein sternförmiges Sechseck ist, wodurch es möglich ist, Licht nach außen abzustrahlen.

**2.** Lichtreaktives Verfahren nach Anspruch 1, wobei die den Flüssigphasenabschnitt (11) bildende Flüssigkeit eine nicht brennbare Flüssigkeit ist.

**3.** Lichtreaktives Verfahren nach Anspruch 1 oder 2, wobei die den Flüssigphasenabschnitt (11) bildende Flüssigkeit Wasser ist.

**4.** Lichtreaktives Verfahren nach einem der Ansprüche 1 bis 3, wobei der zweite optisch transparente Behälter (10) mindestens entlang eines Außenumfangs des ersten optisch transparenten Behälters (8) angeordnet ist.

**5.** Lichtreaktives Verfahren nach einem der Ansprüche 1 bis 4, wobei mindestens einer des ersten optisch transparenten Behälters (8) und des zweiten optisch transparenten Behälters (10) aus einem Material mit einem Brechungsindex von 1,4 oder mehr gebildet ist.

**6.** Lichtreaktives Verfahren nach einem der Ansprüche 1 bis 5, wobei mindestens einer des ersten optisch transparenten Behälters (8) und des zweiten optisch transparenten Behälters (10) aus einem Glas gebildet ist.

**7.** Lichtreaktives Verfahren nach einem der Ansprüche 1 bis 6, wobei der Licht emittierende Körper (11) mit dem Gasphasenabschnitt (9) bedeckt ist, und der Gasphasenabschnitt (9) aus einem Gas mit einer Sauerstoffkonzentration von 1% oder weniger gebildet ist.

**8.** Lichtreaktives Verfahren nach Anspruch 7, wobei der Gasphasenabschnitt (9) aus einem Inertgas gebildet ist.

**9.** Lichtreaktives Verfahren nach Anspruch 7 oder 8, wobei der Gasphasenabschnitt (9) aus Stickstoff gebildet ist.

**10.** Lichtreaktives Verfahren nach Anspruch 1, wobei ein Cycloalkanonoxim hergestellt wird, indem eine Lichtbestrahlung des Cycloalkans und eines Photonitrosierungsmittel durchgeführt wird.

**11.** Lichtreaktives Verfahren nach Anspruch 10, wobei das Cycloalkanonoxim Cyclohexanonoxim oder Cyclododecanonoxim ist.

**12.** Lichtreaktives Verfahren nach Anspruch 10 oder 11, wobei das Photonitrosierungsmittel Nitrosylchlorid oder Trichlornitrosomethan ist.

**13.** Verfahren zur Herstellung eines Lactams, **dadurch gekennzeichnet, dass** ein nach dem lichtreaktiven Verfahren nach einem der Ansprüche 10 bis 12 hergestelltes Cycloalkanonoxim verwendet wird.

**Revendications**

**1.** Procédé de photoréaction utilisant un dispositif de photo-irradiation (1), dans lequel un premier récipient optiquement transparent (8) pour recouvrir un corps électroluminescent (6) est prévu, et à l'extérieur de celui-ci, une section en phase liquide (11) formée d'un liquide ayant un indice de réfraction plus proche de celui dudit premier récipient optiquement transparent (8) que de celui d'un gaz formant une section en phase gazeuse (9) à l'intérieur dudit premier récipient optiquement transparent (8), et un deuxième récipient optiquement transparent (10) pour recouvrir ladite section en phase liquide (11) sont prévus, dans lequel
le dispositif de photo-irradiation (1) est inséré dans une cuve de réaction (2), la cuve de réaction (2) comprenant en outre un liquide de réaction (3), où ledit liquide comprend un cycloalcane,
**caractérisé en ce que** le dispositif de photo-irradiation (1) utilise des diodes électroluminescentes (5) comme source de lumière et **en ce que** le corps électroluminescent (6) est muni desdites diodes électroluminescentes (5),
dans lequel le corps électroluminescent (6) comprend une pluralité de diodes électroluminescentes (5) montées sur un substrat plan (12), et
dans lequel le substrat plan (12) est fixé à chaque surface externe d'un corps de structure (13) dont la forme de section transversale est un hexadécagone en forme d'étoile, moyennant quoi il est possible d'émettre de la lumière vers l'extérieur.

**2.** Procédé de photoréaction selon la revendication 1, dans lequel ledit liquide formant ladite section en phase liquide (11) est un liquide ininflammable.

**3.** Procédé de photoréaction selon la revendication 1 ou 2, dans lequel ledit liquide formant ladite section en phase liquide (11) est de l'eau.

**4.** Procédé de photoréaction selon l'une quelconque des revendications 1 à 3, dans lequel ledit deuxième récipient optiquement transparent (10) est disposé au moins le long d'une circonférence externe dudit premier récipient optiquement transparent (8).

**5.** Procédé de photoréaction selon l'une quelconque des revendications 1 à 4, dans lequel au moins l'un dudit premier récipient optiquement transparent (8) et dudit deuxième récipient optiquement transparent (10) est formé d'un matériau ayant un indice de réfraction supérieur ou égal à 1,4.

**6.** Procédé de photoréaction selon l'une quelconque des revendications 1 à 5, dans lequel au moins l'un dudit premier récipient optiquement transparent (8) et dudit deuxième récipient optiquement transparent (10) est formé d'un verre.

**7.** Procédé de photoréaction selon l'une quelconque des revendications 1 à 6, dans lequel ledit corps électroluminescent (11) est recouvert par ladite section en phase gazeuse (9), et ladite section en phase gazeuse (9) est formée d'un gaz ayant une concentration en oxygène inférieure ou égale à 1%.

**8.** Procédé de photoréaction selon la revendication 7, dans lequel ladite section en phase gazeuse (9) est constituée d'un gaz inerte.

**9.** Procédé de photoréaction selon la revendication 7 ou 8, dans lequel ladite section en phase gazeuse (9) est constituée d'azote.

**10.** Procédé de photoréaction selon la revendication 1, dans lequel une cycloalcanone oxime est produite en effectuant une photo-irradiation dudit cycloalcane et d'un agent de photo-nitrosation.

**11.** Procédé de photoréaction selon la revendication 10, dans lequel ladite cycloalcanone oxime est une cyclohexanone oxime ou une cyclododécanone oxime.

12. Procédé de photoréaction selon la revendication 10 ou 11, dans lequel ledit agent de photo-nitrosation est le chlorure de nitrosyle ou le trichloronitrosométhane.

13. Procédé de production d'un lactame **caractérisé par** l'utilisation d'une cycloalcanone oxime produite par le procédé de photoréaction selon l'une quelconque des revendications 10 à 12.

FIG. 1

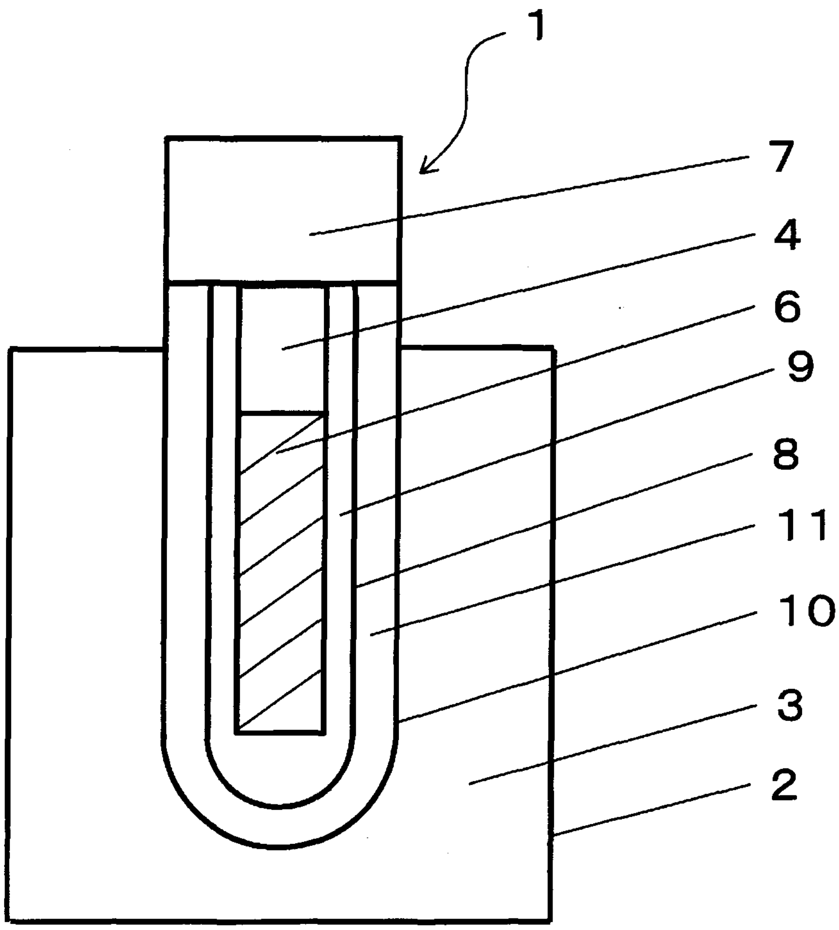


1 : Photoirradiation device
2 : Reaction vessel
3 : Reaction liquid
4 : Power supply unit
6 : Light-emitting body
7 : Lid
8 : First optically transparent container
9 : Gas phase section
10 : Second optically transparent container
11 : Liquid phase section

FIG. 2

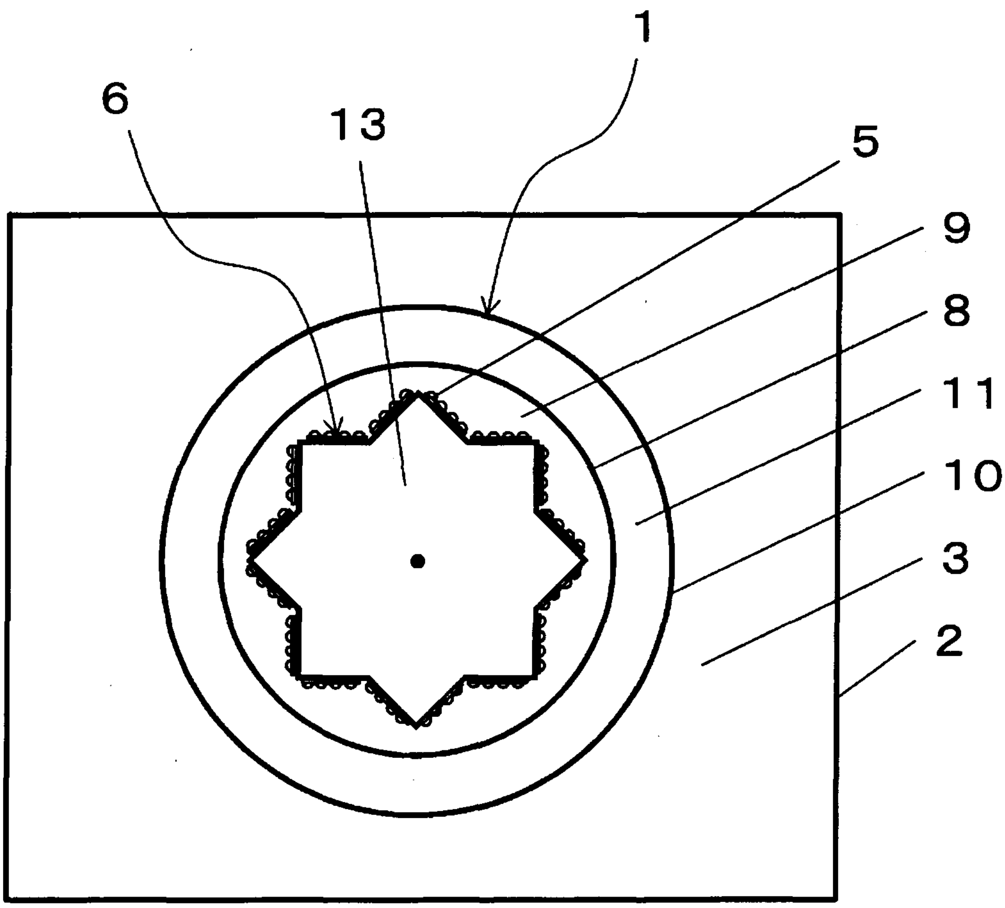


1 : Photoirradiation device
2 : Reaction vessel
3 : Reaction liquid
5 : Light-emitting diode
6 : Light-emitting body
8 : First optically transparent container
9 : Gas phase section
10 : Second optically transparent container
11 : Liquid phase section
13 : Structural body

FIG. 3

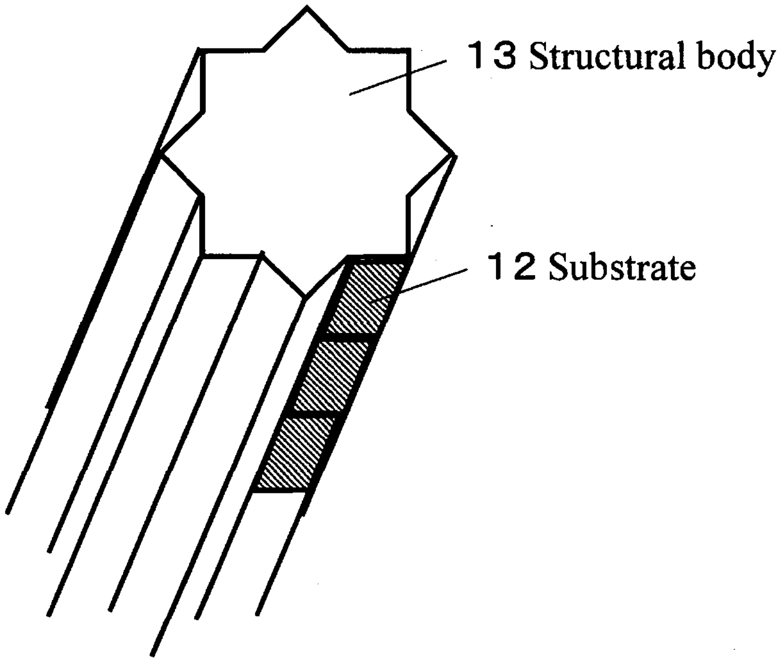
13 Structural body
12 Substrate

FIG. 4

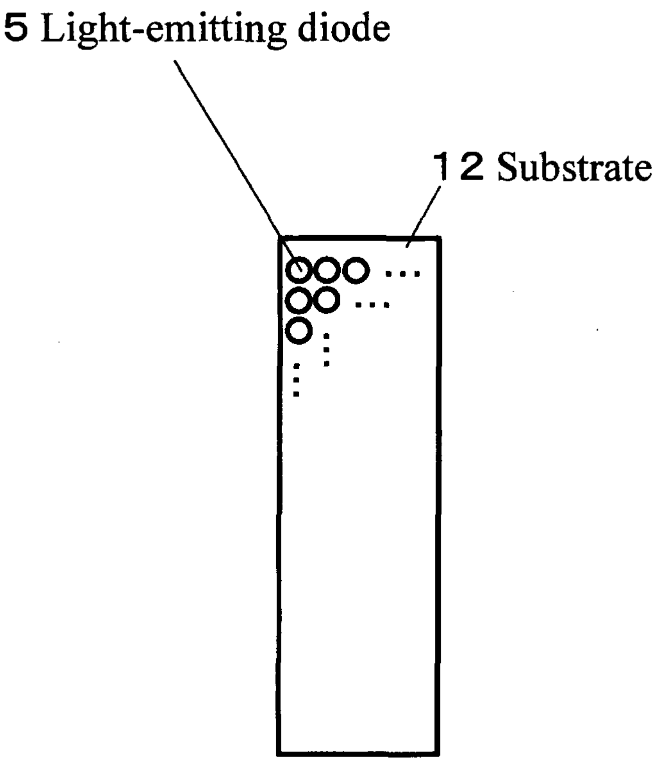
5 Light-emitting diode
12 Substrate

# FIG. 5

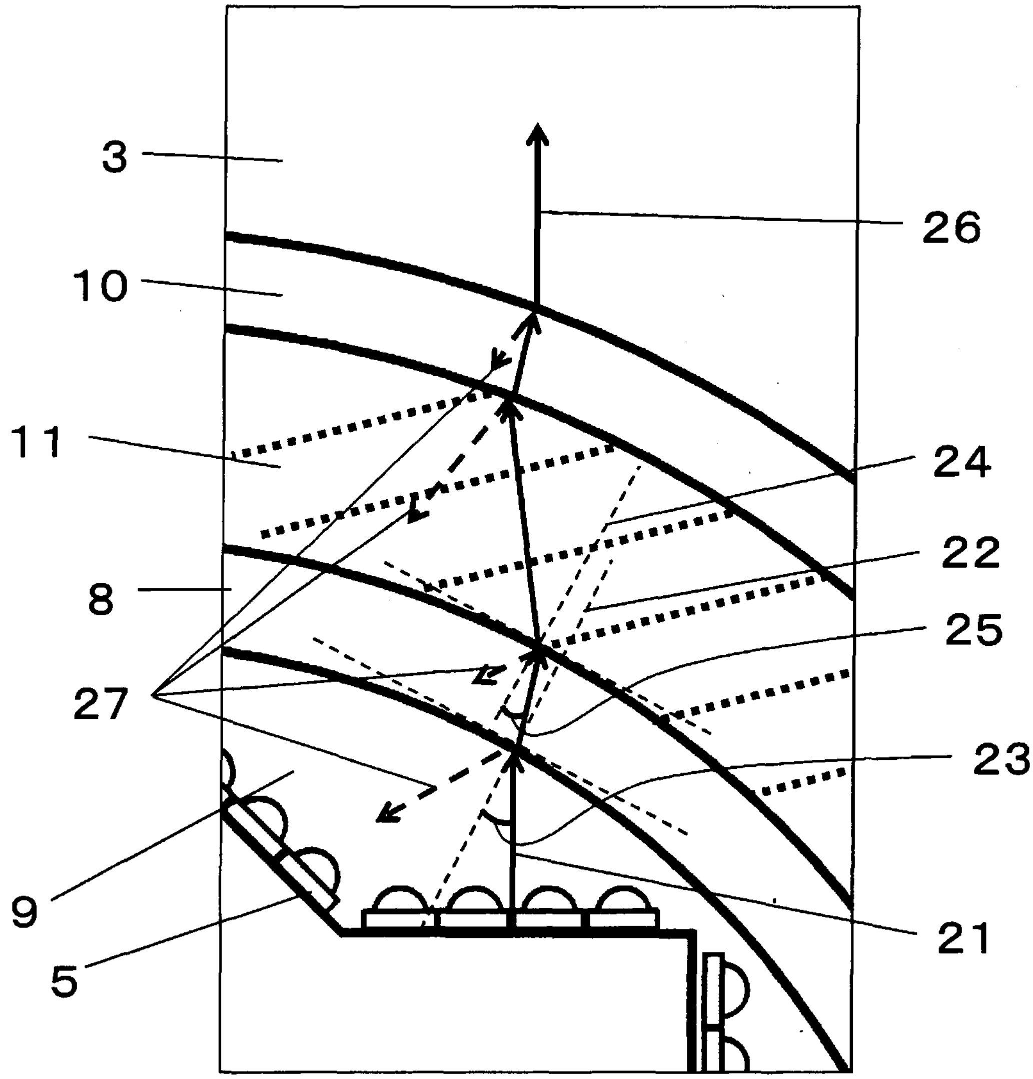


3 : Reaction liquid
5 : Light-emitting diode
8 : First optically transparent container
9 : Gas phase section
10 : Second optically transparent container
11 : Liquid phase section
21 : Optical axis (incident light)
22 : Normal line of inner surface of container
23 : Incident angle to inner surface of container
24 : Normal line of outer surface of container
25 : Incident angle to outer surface of container
26 : Transmitted light
27 : Reflected light

# FIG. 6

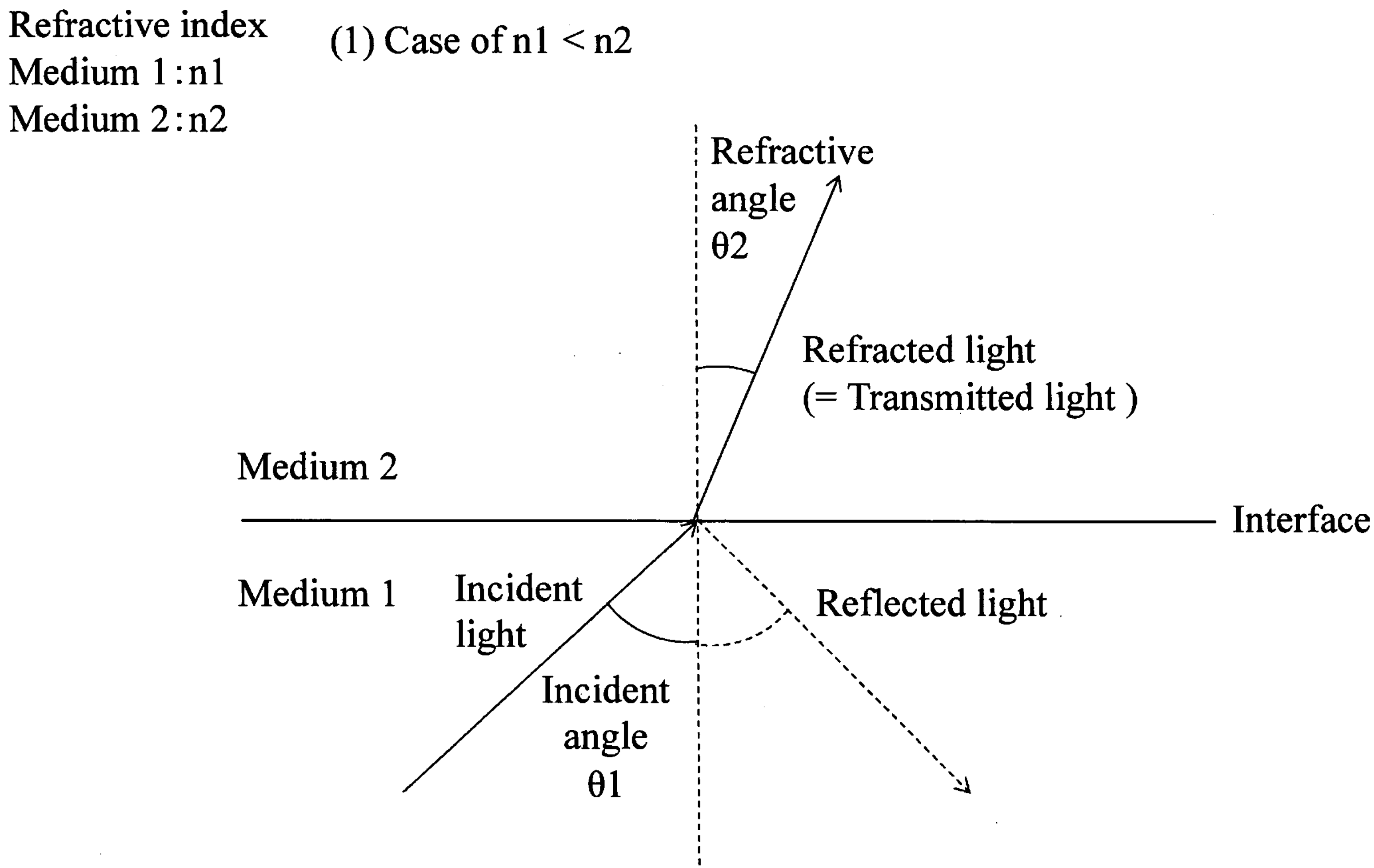
Refractive index
Medium 1 : n1
Medium 2 : n2
(1) Case of n1 < n2
Refractive angle θ2
Refracted light (= Transmitted light )
Medium 2
Interface
Medium 1
Incident light
Reflected light
Incident angle θ1

# FIG. 7

Refractive index
Medium 1 : n1
Medium 2 : n2

(2) Case of n1 > n2

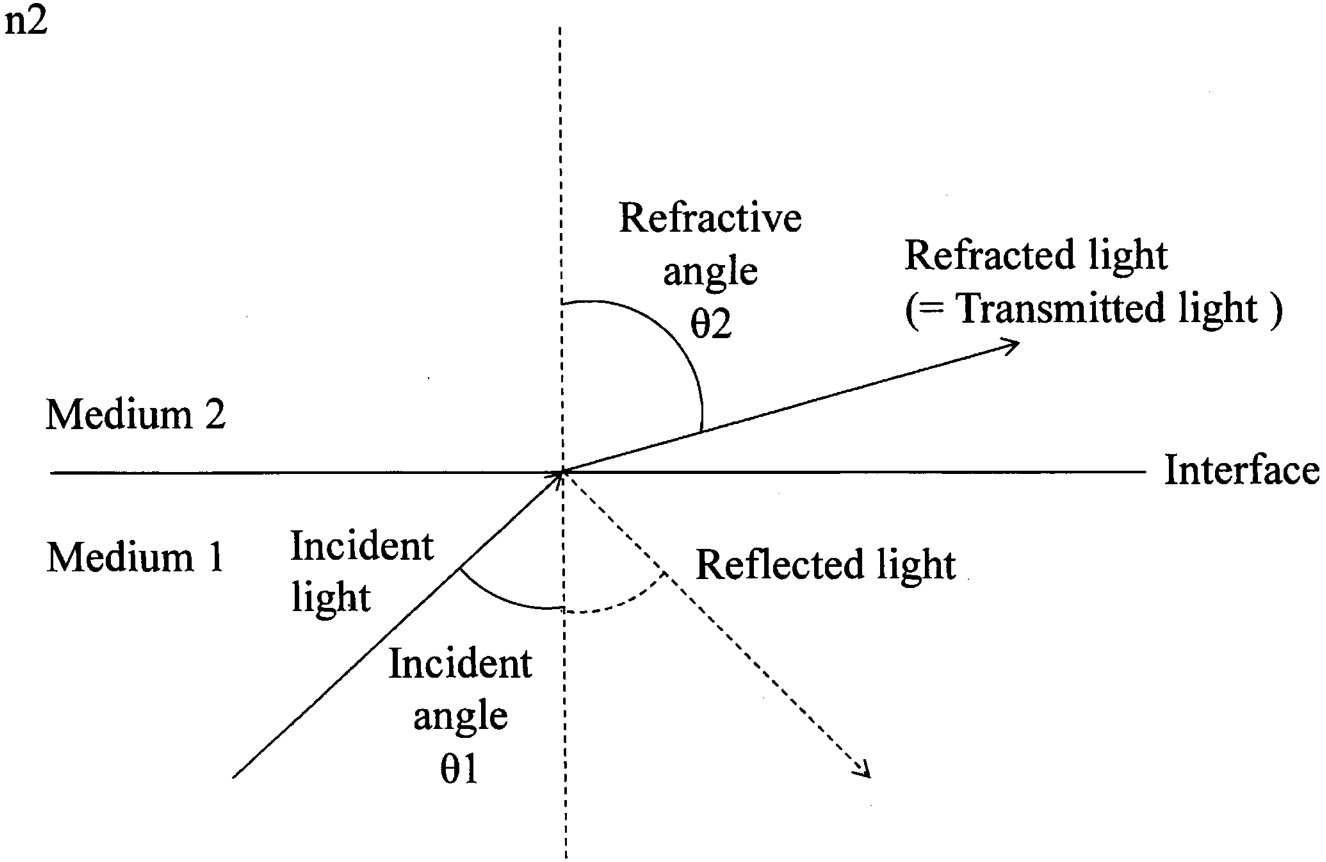

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010006775 A
- JP 2010006776 A
- JP 2013200944 A